# EUROPÄISCHE PATENTANMELDUNG

(11) **EP 2 014 263 A1**
(43) Veröffentlichungstag der Anmeldung: **14.01.2009**
(21) Anmeldenummer: 08156011.2
(22) Anmeldetag: 09.05.2008
(51) Int. Cl.: A61F 2/42

(54) **Gelenkteilprothese mit einer Verdrehsicherung**

(30) Priorität: 09.07.2007 DE 202007009620 U
(71) Anmelder: Zrinski AG, 78573 Wurmlingen (DE)
(72) Erfinder: Lindner, Nicola, 78573, Wurmlingen (DE); Eckhof, Stephan, 78604, Rietheim-Weilheim (DE); Feldhaus, Thomas, 78532, Tuttlingen (DE)
(74) Vertreter: Muri, Peter

(57) **Zusammenfassung**

Die Erfindung bezieht sich auf eine Gelenkteilprothese, insbesondere für ein Fingergelenk, bestehend entweder aus einer proximalen oder distalen Komponente sowie ein von dieser Komponente sich wegweisender in einen Knochen zu lagernden Schaft.

Erfindungsgemäß ist vorgesehen, dass der Schaft (4) zumindest mit einem finnenartigen Anti-Verdrehelement (8) ausgebildet ist, das auf der Mantelfläche (6) des Schafts (4) angeordnet ist und sich radial von dieser Mantelfläche wegerstreckt, wobei sich das Anti-Verdrehelement (8) zumindest zum Teil auch in Längsrichtung des Schaftes (4) erstreckt.

## Beschreibung

### Technisches Gebiet

Die Erfindung bezieht sich auf eine Gelenkteilprothese, insbesondere für ein Fingergelenk. Sie besteht entweder aus einer proximalen oder einer distalen Komponente sowie einem von dieser Komponente sich wegerstreckenden in einem Knochen zu lagernden Schaft.

### Stand der Technik

Künstliche Fingergelenke bestehen im Wesentlichen aus zwei Elementen, nämlich einer proximalen und einer distalen Komponente. Eine Komponente weist einen konvexen Gelenkkopf auf, die mit dem anderen Teil, das eine konkave Gelenkschale zeigt, zusammenwirkt.

Künstliche Fingergelenke werden zwischen dem Mittelhandknochen und dem Fingerknochen oder zwischen einzelnen Fingerknochen eingesetzt. Solche Gelenke müssen dann eingesetzt werden, wenn degenerative Gelenkkrankheiten, wie Osteoarthritis, posttraumatische Arthritis oder Rheumatuid-Arthritis der jeweiligen Gelenke vorliegen. Eine Alternative ist, die Fingergelenke zu versteifen. Eine andere Alternative, die die Mobilität der einzelnen Fingerglieder erhält, ist der totale Gelenkersatz.

Aus dem Stand der Technik, insbesondere aus der EP 1203569 A (FINSBURY (DEVELOPMENT) LIMITED LEATHERHEAD) 03.11.2000 sind Fingergelenkimplantate in der Ausführung sogenannter PIP-Schaftimplantate bekannt. Sie weisen eine proximale oder distale Komponente auf, die entsprechend zusammenwirken und jeweils einen von diesen Komponenten wegweisenden Schaft aufweisen. Der Schaft ist in der Regel konisch ausgebildet und wird in das Knochenmark eines Knochens eingeführt. Um die Einführung zu ermöglichen, wird mit einem Räumwerkzeug das Knocheninnere teilweise ausgeräumt und mit einem hammerartigen Instrument das Implantat in den Knochen eingeschlagen. Die Fixierung erfolgt derart, dass der Durchmesser der ausgeräumten Bohrung kleiner ist als der Außendurchmesser des Schaftes, so dass eine Art Presspassung zwischen dem Schaft und dem Inneren des Knochens entsteht.

Das Implantat besteht selbst aus einem Material mit einem knochenähnlichen Elastizitätsmodul. Dieses knochenähnliche Elastizitätsmodul vermeidet das sogenannten Stress-Shilding und fördert somit den Knochenaufbau. Dadurch wird zusätzlich die Fixierung des Implantats im Markraum des Knochens verbessert.

### Nachteile des Standes der Technik

Produkte gemäss dem Stand der Technik weisen Nachteile auf, wie Implantatlockerung aufgrund mangelnder Fixierung und mangelnder Verbindung mit dem Knochen sowie hohe Verschleißwerte, wodurch entsprechender Abrieb entsteht. Die schaftähnliche Konstruktion innerhalb des Markraumes verschafft sich bei Lockerung ausreichend Platz, so dass ein einmal fixiertes Implantat nicht mehr funktionsgerecht vorliegt. Die Folgen davon sind Schmerzen, die durch Migration oder sogar Brüche hervorgerufen werden.

Weiterhin besteht der Nachteil der Ausführungen gemäss dem Stand der Technik darin, dass die einzelnen Gelenkteilprothesen nicht verdrehsicher angeordnet werden können.

### Aufgabe der Erfindung

Daher ist es Aufgabe der Erfindung, eine Gelenkteilprothese, insbesondere für Fingergelenke derart weiterzubilden, dass die Wahrscheinlichkeit einer Lockerung des Implantats im Gegensatz zum Stand der Technik verringert wird.

### Lösung der Aufgabe

Kerngedanke der Lösung der Aufgabe ist es, den Schaft mit flügelartigen bzw. finnenartigen Ausbildungen zu versehen, so dass die für die Herstellung und das Ausräumen des Markraums des Knochens vorteilhafte rotationssymmetrische Form erhalten bleibt, aber trotzdem eine Verdrehsicherung vorgesehen ist.

### Vorteile der Erfindung

Einer der wesentlichen Vorteile der Erfindung ist es, dass aufgrund der erfinderischen Ausgestaltung der Gelenkteilprothese es nicht mehr notwendig ist, durch schlagende bzw. hämmernde Bearbeitung den Markraum des Knochens, in den das Implantat zu platzieren ist, freizuräumen. Trotzdem ist die Gelenkteilprothese einfach und effizient fixierbar. Da der Schaft der Gelenkteilsprothese rotationssymmetrisch ist, genügt es, durch drehende Bewegung den Markraum des Knochens auszuräumen. Dadurch werden Schäden insbesondere an benachbarten Gelenken, hervorgerufen durch den an sich für das Ausräumen notwendigen Hammervorgang, vermieden.

Einer der weiteren wesentlichen Vorteil der Erfindung ist es, dass die Gelenkteilprothese mit ihrem Schaft in den Markraum eines Knochens eingefügt werden kann und dort auch drehfest fixiert ist, ohne dass weitere Schritte notwendig sind.

Die Erfindung sieht vor, dass auf der Mantelfläche des Schaftes der Gelenkteilprothese Anti-Verdreheinrichtungen vorgesehen sind. Eine Anti-Verdreheinrichtung umfasst mindestens ein finnenartiges Element, welches an der Mantelfläche des Schaftes der Gelenkteilprothese angeordnet ist und sich in Längserstreckung des Schaftes ausdehnt. Dabei ist es vorzugsweise schmal und vorteilhafterweise zum Schaft hin etwas dicker ausgebildet. Dies bringt den Vorteil mit sich, dass zum einen auftretende Drehmomente / Torsionen von dem Anti-Verdrehelement aufgenommen werden können. Zum anderen hat es den Vorteil, aufgrund dessen, dass die freien Enden schmäler ausgebildet sind, ein einfaches Einschieben in den vorbereiteten ausgeräumten Markraum möglich ist.

Eine bevorzugte Ausführungsform dieser Anti-Verdrehsicherung sieht vor, dass sie von dem freien Ende ausgehend in Richtung der distalen oder proximalen Komponente in ihren Ausladungen zunehmen, so dass beim Einführen des Implantats eine Verdrehung noch möglich ist und je weiter das Implantat in den Markraum hineingeführt wird, desto eher die Verdrehung eingeschränkt ausgebildet ist.

Um solche Anti-Verdreheinrichtungen zusammen mit dem Schaft einer Komponente installieren zu können, ist vorgesehen, den Hohlraum entsprechend zu räumen. Neben den Abmessungen des Schaftes werden durch eine Lehre die seitlich abstehenden Räume ebenfalls geräumt, so dass das Implantat passgenau eingesetzt werden kann. Die Räumung erfolgt vorzugsweise durch drehende und oszillierende Bewegungen, so dass keine hämmernde und gelenkbelastende Bewegungen notwendig sind.

Die Gelenkteilprothese besteht bei einem Ausführungsbeispiel aus einem an sich aus dem Stand der Technik bekannten Material.

Vorzugsweise bilden die Anti-Verdrehelemente mit dem Schaft ein einstückiges Teil.

Eine bevorzugte Ausführungsform sieht vor, dass sich der Abstand von der Mantelfläche des Schaftes zum freien Ende des Anti-Verdrehelements in Richtung freies Ende des Schaftes hin verjüngt.

Eine weitere Ausbildung sieht vor, dass die finnenartigen Elemente sich nur teilweise über die Längserstreckung des Schafts ausdehnen. Vorteilhafterweise sind sie nur abschnittsweise ausgebildet.

Alternativen sehen vor, dass, um insbesondere das knochenähnliche Elastizitätsmodul zu erreichen, ein Kunststoff verwendet werden kann. Dies bringt auch insbesondere den Vorteil mit sich, dass die Gelenkteilprothesen mittels Spritzgussverfahren hergestellt werden können.

Weitere vorteilhafte Ausgestaltungen gehen aus der nachfolgenden Beschreibung, den Zeichnungen sowie den Ansprüchen hervor.

### Zeichnung

Es zeigt:

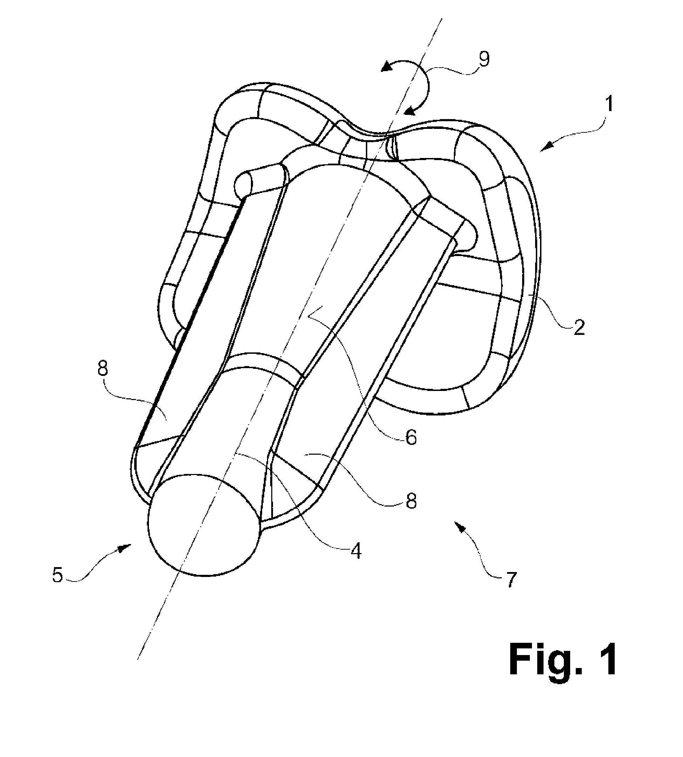

Fig. 1 eine perspektivische Ansicht auf die erfindungsgemässe Gelenkteilprothese mit proximaler Komponente, mit einer angeordneten Verdrehsicherung.

### Beschreibung eines Ausführungsbeispiels

In Figur 1 ist eine Ausführungsform der erfindungsgemässen Ausführungsform der Erfindung gezeigt. Die dort dargestellte Gelenkteilprothese 1 besteht umfasst eine proximale Komponente 2, von der sich aus rückwärtig ein Schaft 4 erstreckt. Der Schaft 4 erstreckt sich nahezu senkrecht weg von der proximalen Komponente 2 und weist bei dem hier dargestellten Ausführungsbeispiel eine Verjüngung zu seinem freien Ende 5 hin auf. Er ist im Querschnitt rund ausgestaltet. Der runde Querschnitt bringt den Vorteil, dass diese Ausführungsform einfacher und kostengünstiger herstellbar ist, als beispielsweise eine ovale oder rechteckige Form.

Das freie Ende 5 des Schaftes 4 ist vorzugsweise kugelartig ausgestaltet, so dass keine scharfen Kanten entstehen können, die beim Einsetzen der Prothese etwaiges Mark des Knochens verletzen können.

Zudem ist die Wandung des Schaftes 4 insbesondere im Bereich des freien Endes 5 rund ausgestaltet.

An der Mantelfläche 6 ist die erfindungsgemässe Anti-Verdreheinrichtung 7 angeordnet. Diese Anti-Verdreheinrichtung 7 dient dazu, eine einmal in einem Markraum, der vorzugsweise zumindest nahezu den Querschnitt einnimmt wie der Schaft 4 zusammen mit der Anti-Verdreheinrichtung 7 ausgestaltet ist, eingeführte Gelenkteilprothese 1 zu fixieren, derart, dass ein Verdrehen der Gelenkteilprothese 1 innerhalb des Markraumes nicht mehr möglich ist.

Die Anti-Verdreheinrichtung 7 besteht mindestens aus einem Anti-Verdrehelement 8, das sich in Längserstreckung des Schaftes 4 ausbildet und finnenartige Gestaltung hat.

Insbesondere im Bereich des freien Endes 5 des Schaftes 4 ist vorgesehen, das Anti-Verdrehelement 8 in Bezug zu seinem Abstand zur Mantelfläche des Schaftes 4 sehr geringfügig auszubilden, so dass beim Einführen noch ein entsprechendes Positionieren innerhalb des Markraumes möglich ist. Je tiefer die Gelenkteilprothese 1 in den Markraum eingeführt wird, desto mehr durchdringen die finnenartigen Anti-Verdreheinrichtung 7 den Markraum und schneiden und drücken sich in das Mark des Knochens ein. Eine entsprechende Keilwirkung entsteht.

Das Anti-Verdrehelement 7 verhindert die rotatorische Bewegung der Gelenkteilprothese 1 in oder gegen Pfeilrichtung 9. Sie dient zumindest nur geringfügig dazu, ein Fixieren der Gelenkteilprothese 1 in Rotationsrichtung aber auch in Längsrichtung zu erwirken.

Die Erfindung ist sowohl für Gelenkteilprothesen im distalen oder proximalen Bereich anzuwenden. Ebenso ist eine Anwendung bei allen Arten von Prothesen denkbar, die eine schaftartige Ausbildung aufweisen und es zu verhindern gilt, dass eine rotatorische Bewegung der Gelenkteilprothesen möglich ist.

### Bezugszeichenliste

- 1: Gelenkteilprothese
- 2: proximale Komponente
- 3: -
- 4: Schaft
- 5: freies Ende
- 6: Mantelfläche
- 7: Anti-Verdreheinrichtung
- 8: Anti-Verdrehelement
- 9: Pfeilrichtung

## Patentansprüche

1. Gelenkteilprothese, insbesondere für ein Fingergelenk, bestehend entweder aus einer proximalen oder distalen Komponente sowie ein von dieser Komponente sich wegweisender in einen Knochen zu lagernden Schaft, **dadurch gekennzeichnet, dass** der Schaft (4) zumindest mit einem finnenartigen Anti-Verdrehelement (8) ausgebildet ist, das auf der Mantelfläche (6) des Schafts (4) angeordnet ist und sich radial von dieser Mantelfläche wegerstreckt, wobei sich das Anti-Verdrehelement (8) zumindest zum Teil auch in Längsrichtung des Schaftes (4) erstreckt.

2. Gelenkteilprothese nach Anspruch 1, **dadurch gekennzeichnet, dass** Anti-Verdrehelement (8) und Schaft (4) ein einstückiges Teil bilden.

3. Gelenkteilprothese nach mindestens einem der vorhergehenden Ansprüche, **dadurch gekennzeichnet, dass** das Anti-Verdrehelement (8) zum Schaft hin dicker ausgebildet ist.

4. Gelenkteilprothese nach mindestens einem der vorhergehenden Ansprüche, **dadurch gekennzeichnet, dass** sich der Abstand von der Mantelfläche (6) des Schaftes (4) zum freien Ende des Anti-Verdrehelements (8) in Richtung freies Ende (5) des Schaftes (4) hin verjüngt.

5. Gelenkteilprothese nach mindestens einem der vorhergehenden Ansprüche, **dadurch gekennzeichnet, dass** der Schaft (4) aus Kunststoff besteht.

6. Gelenkteilprothese nach mindestens einem der vorhergehenden Ansprüche, **dadurch gekennzeichnet, dass** der Schaft (4) eine raue Oberfläche umfasst.
